# EUROPEAN PATENT SPECIFICATION

(11) **EP 4 175 599 B1**
(45) Date of publication and mention of the grant of the patent: **30.10.2024**
(21) Application number: 21752777.9
(22) Date of filing: 24.06.2021
(51) Int. Cl.: A61F 13/38, A61B 10/00, A61B 10/02, B01L 3/00

(54) **SPECIMEN COLLECTION AND STORAGE FOR POOL TESTING AND SIMILAR**
PROBENENTNAHME UND LAGERUNG FÜR POOLTESTS UND DERGLEICHEN
COLLECTE ET STOCKAGE D'ÉCHANTILLONS POUR TESTS GROUPÉS ET SIMILAIRES

(30) Priority: 15.07.2020 US 202063052062 P; 06.01.2021 US 202163134209 P
(43) Date of publication of application: 10.05.2023
(73) Proprietor: Fresenius Medical Care Holdings, Inc., Waltham, MA 02451 (US)
(72) Inventor: KOTANKO, Peter, Waltham, MA 02451 (US); GROBE, Nadja, Waltham, MA 02451 (US); WANG, Xiaoling, Waltham, MA 02451 (US); CHERIF, Alhaji, Waltham, MA 02451 (US); GARBACCIO, Mia, G., Waltham, MA 02451 (US)
(74) Representative: Smith, Jeremy Robert
(86) International application number: PCT/US2021/038808
(87) International publication number: WO 2022/015479

(56) References cited:
- CN-A- 110 547 829
- JP-U- 3 159 968
- US-A1- 2019 033 175

## Description

### CROSS-REFERENCE TO RELATED APPLICATIONS

This application claims priority to U.S. Provisional Patent Application No. 63/052,062 filed on July 15, 2020 and U.S. Provisional Patent Application No. 63/134,209 filed on January 6, 2021.

### FIELD

The present disclosure generally relates to specimen collection techniques for pool testing and the like. For example, the present disclosure includes devices, systems, kits, and methods for collecting, storing, and transporting specimens for pool testing and the like.

### BACKGROUND

Pool testing refers to a technique where a specimen (e.g., a biological specimen such as a nasopharyngeal specimen collected via a swab) is collected from a plurality of subjects (e.g., humans, animals, plants, environmental samples from bodies of water, and the like) in a batch, which is referred to as a "pool," and the collective pool is then tested. If the pool tests positive, the individual samples are then tested to identify the subject(s) that contributed to the positive pool test. While the theory of pool testing is generally well understood (*see, e.g.,* Alhaji Cherif, et al., "Simulation of Pool Testing to Identify Patients With Coronavirus Disease 2019 Under Conditions of Limited Test Availability," JAMA Network Open, doi: 10.1001/jamanetworkopen.2020.13075 (June 23, 2020)), the practical execution of pool testing can pose challenges, e.g., due to dilution effects (which can particularly be problematic in high-sensitivity reverse transcription polymerase chain reaction (RT-PCR) tests) or a need for repeated collection using a conventional swab.

There remains a need for improved specimen collection techniques, e.g., for pool testing. There further remains a need for improved techniques related to collecting, storing, and transporting specimens for pool testing and the like.

US 2019/0033175 describes a device for collecting, transferring and storing samples of biological and/or chemical material. The device includes a support body extending along a main longitudinal direction between a first and a second end opposite each other and a collecting portion engaged with one of said ends of the support body and suitable for collecting an amount of a sample of biological and/or chemical material. The support body is defined by at least a first sub-body and a second sub-body extending longitudinally and configured to be selectively engaged and separated with and from each other. The support body is configured to operate between an assembled condition, wherein the first and the second sub-body are engaged with each other, and a separated condition, wherein the first and the second sub-body are separated and bear, respectively, a first and a second sub-collecting portion of the collecting portion.

JP 3159968 describes a specimen-collecting rod that allows the same specimen to be used for a plurality of examinations, and a container equipped with the specimen-collecting rod. A plurality of rods of the sampling rod are tied together so that the rod tip sampling portion can be divided into the same number as the rods, the specimen sampling rod is fitted into the plug, and the specimen sampling rod is divided into front and rear parts of the plug. A tubular container capable of separately accommodating the specimen-picking rods is fitted.

CN 110547829 describes a medical care inspection sampling apparatus, in particular to a disposable sterile sampling swab. The disposable sterile sampling swab consists of a fixing cap, a detachable sampling swab a, a detachable sampling swab b, a C-shaped clip and a sleeve pipe, wherein the head of the detachable sampling swab a and the head of the detachable sampling swab b are integrally fixed through the C-shaped clip; and the fixing cap is fixedly connected with the handle parts of the detachable sampling swab a and the detachable sampling swab b which are combined, and is placed in the sleeve pipe.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

The present teachings generally include devices, systems, kits, and methods for collecting, storing, and transporting specimens, e.g., in the context of pool testing. For example, the present teachings may include a compound swab featuring a first swab and a second swab, where the first swab can be detached for pool testing and the second swab can be preserved for testing based on results of the pool test. The present teachings may also or instead include a container having a separator structurally configured to separate swabs of a compound swab. Further, the present teachings may include a swab having a suction enhancer to promote the capture of a specimen on a collection tip thereof. The present teachings may also or instead include a receptacle that can house a plurality of swabs and sever at least a portion of the collections tips thereof, e.g., for pool testing.

Disclosed herein is a device for collecting a specimen for testing which may include: a first swab including a first shaft and a first collection tip disposed on an end of the first shaft, the first collection tip structurally configured to acquire a first portion of a specimen thereon; a second swab including a second shaft and a second collection tip disposed on an end of the second shaft, the second collection tip structurally configured to acquire a second portion of the specimen thereon; one or more first couplers disposed along the first swab; and one or more second couplers disposed along the second swab, where the one or more first couplers are removably engageable with the one or more second couplers to establish a compound swab to collect the specimen on each of the first collection tip and the second collection tip, and where disengagement of the one or more first couplers and the one or more second couplers permits separate testing of the specimen.

Implementations may include one or more of the following features. The first swab may be used in a pool testing procedure, and the second swab may be preserved for testing of the specimen based on results of the pool testing procedure. One or more of the first couplers and the second couplers may include at least one of a projection and a void. One or more of the first couplers and the second couplers may include a hook and loop fastener. One or more of the first couplers may be disposed on the first shaft, and one or more of the second couplers may be disposed on the second shaft. At least one of the first couplers may be disposed on the first collection tip, and at least one of the second couplers may be disposed on the second collection tip. The first collection tip and the second collection tip may be made of one or more of polyester, rayon, and flocked nylon. At least a portion of one or more of the first collection tip and the second collection tip may be removable from its corresponding shaft. The first shaft and the second shaft may be selectively flexible to conform to at least a portion of a nasal passage of an animal. The first shaft and the second shaft may be made of one or more of a plastic material and a metal. The specimen may be a nasopharyngeal specimen. The device may be structurally configured for collection of the nasopharyngeal specimen from a surface of respiratory mucosa of an animal.

In an aspect of the presently claimed invention, a method of pool testing includes collecting a specimen using a compound swab, the compound swab including: a first swab including a first shaft and a first collection tip disposed on an end of the first shaft; and a second swab coupled to the first swab, the second swab including a second shaft and a second collection tip disposed on an end of the second shaft. The method also includes: separating the first swab and the second swab; combining the first swab with a plurality of different swabs for pool testing the specimen with other, different specimens for presence or absence of a component; and, based on results of the pool testing, testing the second swab for presence or absence of the component.

Also disclosed herein is a device for collecting a specimen for testing which may include: a first swab including a first shaft and a first collection tip disposed on a first end of the first shaft, the first collection tip structurally configured to acquire a first portion of a specimen thereon; a second swab including a second shaft and a second collection tip disposed on a first end of the second shaft, the second collection tip structurally configured to acquire a second portion of the specimen thereon; and a common shaft engaged with a second end the first shaft and a second end of the second shaft, where engagement of the second ends of each of the first shaft and the second shaft to the common shaft define a first breakpoint structurally configured for separating one or more of the first swab and the second swab from the common shaft.

Implementations may include one or more of the following features. The device may further include a channel disposed between a majority of the first shaft and the second shaft. The channel may narrow adjacent to the common shaft. The device may further include a collar disposed between the common shaft and the second ends of each of the first shaft and the second shaft. The first breakpoint may be the only breakpoint for the device. The device may further include a plurality of breakpoints including at least the first breakpoint and a second breakpoint. The first breakpoint may include a first coupler engaged to each of the first swab and the second swab, where the second breakpoint may include a second coupler engaged to each of the first swab and the second swab. The first coupler may include a collar connected to each of the first shaft and the second shaft. The second coupler may include a linkage that connects at least a portion of the first swab to at least a portion of the second swab. The linkage may connect the first collection tip and the second collection tip.

In an aspect of the presently claimed invention, a system for collecting a specimen for testing includes a compound swab including: a first swab including a first shaft and a first collection tip disposed on an end of the first shaft; and a second swab removably coupled to the first swab, the second swab including a second shaft and a second collection tip disposed on an end of the second shaft. The system also includes a container for receiving one or more of the first swab and the second swab, the container including: a first opening leading to a first chamber, the first chamber structurally configured to contain at least a portion of one or more of the first swab and the second swab therein; and a separator disposed within or adjacent to one or more of the first opening and the first chamber, the separator sized and shaped to fit between the first swab and the second swab, where a predetermined force placed on the compound swab directed toward the container when the separator is disposed between the first swab and the second swab separates the first swab and the second swab.

Implementations may include one or more of the following features. The separator may be located on the container such that, upon separation of the first swab and the second swab using the separator, one or more of the first swab and the second swab will be at least partially disposed within the first chamber. The container may include a second opening leading to a second chamber, where the separator may be disposed between a boundary of the first opening and the second opening.

In an aspect of the presently claimed invention, a method for storing a specimen includes: aligning a separator between a first swab and second swab of a compound swab, the separator coupled to a container including a first opening leading to a first chamber, the first chamber structurally configured to contain at least a portion of one or more of the first swab and the second swab therein; and applying a predetermined force against the separator and toward the container with the compound swab thereby separating the first swab and the second swab, where at least the portion of one or more of the first swab and the second swab is disposed within the first chamber upon separation thereof.

Also disclosed herein is a swab for collecting a specimen for testing which may include: a shaft having a first end and a second end, the shaft including a channel disposed therethrough from the second end toward the first end; a collection tip disposed on the second end of the shaft and structurally configured for acquiring a specimen thereon, where the collection tip is permeable to permit a flow of fluid therethrough; and a suction enhancer in fluid communication with the channel, the suction enhancer activatable to establish a flow of fluid through the channel in a first direction from the second end toward the first end thereby establishing suction at the second end of the shaft through the collection tip.

Implementations may include one or more of the following features. The suction enhancer may include a fluid reservoir compressible to a first state, where, when the fluid reservoir is released from the first state, the fluid reservoir draws in fluid from the channel thereby establishing the flow of fluid in the first direction. The suction may be configured to promote capture of the specimen on the collection tip. The swab may further include: a second shaft removably coupled to the shaft, the second shaft having a first end and a second end, and the second shaft including a second channel disposed therethrough; and a second collection tip disposed on the second end of the second shaft and structurally configured for acquiring the specimen thereon, where the second collection tip is permeable to permit a flow of fluid therethrough. The suction enhancer may be in fluid communication with the second channel. The channel and the second channel may be in fluid communication. The swab may further include a second suction enhancer in fluid communication with the second channel. The channel and the second channel may not be in fluid communication with one another.

Also disclosed herein is a method for collecting a specimen for testing which may include inserting a swab within an environment to collect a specimen, where the swab includes: a shaft having a first end and a second end, the shaft including a channel disposed therethrough from the second end toward the first end; and a collection tip disposed on the second end of the shaft and structurally configured to acquire a specimen thereon, where the collection tip is permeable to permit a flow of fluid therethrough. The method may also include activating a suction enhancer in fluid communication with the channel; establishing a flow of fluid through the channel in a first direction from the second end toward the first end; and establishing suction at the second end of the shaft through the collection tip to promote collection of the specimen.

Implementations may include one or more of the following features. The suction enhancer may include a fluid reservoir, and activating the suction enhancer may include releasing the fluid reservoir from a first state where the fluid reservoir is compressed. The method may further include, before insertion of the swab within the environment, compressing the fluid reservoir thereby placing the fluid reservoir in the first state.

Also disclosed herein is a receptacle for collection of a plurality of specimens which may include: a first housing including a plurality of first bores extending through the first housing from a top end to a bottom end, each of the plurality of first bores including a top opening configured to receive a swab therein and a bottom opening configured to project at least a portion of a collection tip of the swab therefrom; and a second housing coupled to the first housing and movable relative to the first housing between a first position and a second position, the second housing including a plurality of second bores each including a collection opening that is aligned with the bottom opening of a first bore and configured to receive at least the portion of the collection tip of the swab when the second housing is in the first position, where movement of the second housing from the first position to the second position removes alignment of the collection opening with the bottom opening of the first bore.

Implementations may include one or more of the following features. When at least the portion of the collection tip of the swab is disposed within one of the plurality of second bores and the second housing is moved from the first position to the second position, an abutment between the first housing and the second housing may be structurally configured to sever at least the portion of the collection tip from the swab. The receptacle may further include a collection volume within the second housing structurally configured to receive at least the portion of the collection tip that is severed from the swab. The receptacle may further include a cutting edge disposed at the abutment between the first housing and the second housing. The second housing may be removable from the first housing. Movement from the first position to the second position may include rotation of the second housing relative to the first housing. The receptacle may further include one or more mechanical stops to limit movement of the second housing relative to the first housing.

Also disclosed herein is a kit for collecting a plurality of specimens which may include: a plurality of swabs, each of the plurality of swabs including a shaft and a collection tip disposed on an end of the shaft; a first housing including a plurality of first bores extending through the first housing from a top end to a bottom end, each of the plurality of first bores including a top opening configured to receive a swab of the plurality of swabs therein and a bottom opening configured to project at least a portion of the collection tip of the swab therefrom; and a second housing coupled to the first housing and movable relative to the first housing between a first position and a second position, the second housing including a plurality of second bores each including a collection opening that is aligned with the bottom opening of a first bore and configured to receive at least the portion of the collection tip of the swab when the second housing is in the first position, where movement of the second housing from the first position to the second position removes alignment of the collection opening with the bottom opening of the first bore.

Implementations may include one or more of the following features. The kit may further include a limiter disposed on one or more of the plurality of swabs, the first housing, and the second housing, where the limiter is structurally configured to limit projection of the portion of the collection tip from the bottom opening of the first bore to a predetermined amount. The limiter may include a projection on the shaft of one or more of the plurality of swabs.

Also disclosed herein is a method for collecting a plurality of specimens which may include: inserting a swab including a shaft and a collection tip into a first bore of a first housing of a receptacle such that at least a portion of the collection tip extends through the first housing and into a second bore of a second housing coupled to the first housing; and moving the second housing relative to the first housing thereby misaligning the first bore from the second bore and severing at least the portion of the collection tip from the swab.

These and other features, aspects, and advantages of the present teachings will become better understood with reference to the following description, examples, and appended claims.

### BRIEF DESCRIPTION OF THE DRAWINGS

The foregoing and other objects, features, and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.
Fig. 1 illustrates a conventional swab of the prior art.
Fig. 2 illustrates a compound swab, in accordance with a representative embodiment.
Fig. 3 illustrates a compound swab in a decoupled state, in accordance with a representative embodiment.
Fig. 4 illustrates a compound swab, in accordance with a representative embodiment.
Fig. 5 illustrates a top view of the compound swab of Fig. 4.
Fig. 6 illustrates a side view of the compound swab of Fig. 4.
Fig. 7 illustrates `Detail B' of Fig. 6.
Fig. 8 illustrates `Detail A' of Fig. 6.
Fig. 9 illustrates a compound swab, in accordance with a representative embodiment.
Fig. 10 illustrates a top view of the compound swab of Fig. 9.
Fig. 11 illustrates a side view of the compound swab of Fig. 9.
Fig. 12 illustrates 'Detail A' of Fig. 11.
Fig. 13 illustrates `Detail B' of Fig. 11.
Fig. 14 illustrates a cross-section of a swab for collecting a specimen for testing, in accordance with a representative embodiment.
Fig. 15 illustrates a cross-section of a swab for collecting a specimen for testing, in accordance with a representative embodiment.
Fig. 16 is a flow chart of a method of pool testing, in accordance with a representative embodiment.
Fig. 17 illustrates a top view of a container for receiving a swab, in accordance with a representative embodiment.
Fig. 18 illustrates a container for receiving a swab, in accordance with a representative embodiment.
Fig. 19 illustrates a top view of a container for receiving a swab, in accordance with a representative embodiment.
Fig. 20 illustrates a cross-section of a receptacle for collection of a plurality of specimens, in accordance with a representative embodiment.
Fig. 21 illustrates a receptacle for collection of a plurality of specimens, in accordance with a representative embodiment.
Fig. 22 illustrates a receptacle for collection of a plurality of specimens, in accordance with a representative embodiment.

### DETAILED DESCRIPTION

The embodiments will now be described more fully hereinafter with reference to the accompanying figures, in which preferred embodiments are shown. The foregoing may, however, be embodied in many different forms and should not be construed as limited to the illustrated embodiments set forth herein. Rather, these illustrated embodiments are provided so that this disclosure will convey the scope to those skilled in the art.

References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "about," "approximately," or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better illuminate the embodiments and does not pose a limitation on the scope of the embodiments. No language in the specification should be construed as indicating any unclaimed element as essential to the practice of the embodiments.

In the following description, it is understood that terms such as "first," "second," "top," "bottom," "up," "down," and the like, are words of convenience and are not to be construed as limiting terms unless specifically stated to the contrary.

In general, the devices, systems, kits, and methods disclosed herein relate to one or more of the collection, storage, transport, and the like, of specimens-e.g., specimens for pool testing. However, it will be understood that aspects of the present teachings may not be limited to simply pool testing, and that many implementations disclosed herein can be adapted for other types of specimen analyses, examination, collection, transport, testing, and so on. Further, although aspects of the present teachings may be useful for the collection of biological specimens such as a nasopharyngeal swab (sometimes referred to as a nasopharyngeal culture) to test a sample of nasal secretions from the back of the nose and throat of a human or other animal (e.g., for suspected cases of whooping cough, diphtheria, influenza, and various types of diseases caused by the coronavirus family of viruses, including SARS, MERS, COVID-19, and the like), other types of specimens may also or instead be utilized with the present teachings including without limitation biological and/or non-biological samples from subjects such as humans, other animals, plants, bodies of water, other environmental samples, or from other environments including animate and inanimate objects, and the like. The specimens themselves may thus include one or more of a virus, an antibody, bacteria, a clinical marker, a substance or organism generally, and the like. The devices, systems, kits, and methods disclosed herein may also or instead be used for hierarchical group testing.

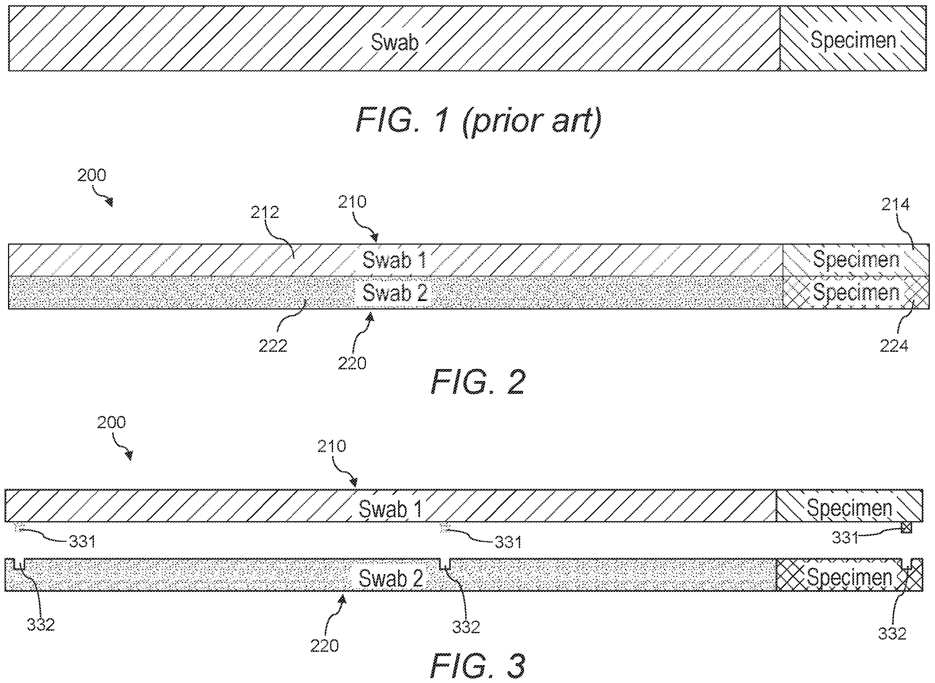

Fig. 1 illustrates a conventional swab of the prior art. The swab may be structurally configured for collecting a sample of nasal secretions from the back of the nose and throat. In this manner, the swab may be structurally configured to be inserted in the nostril and gently moved forward into the nasopharynx, to be rotated for a specified period time to collect secretions. The swab may then be removed and used for testing. The swab may be similar in concept to a cotton swab, including a shaft (e.g., a shaft made of plastic, wood, metal, combinations thereof, and the like) that is covered, at one end, with an absorbent material such as cotton, polyester, flocked nylon (or other flocked material), combinations thereof, and the like. The swab material used for a particular diagnostic application may vary based on the test type.

It will be understood that swabs according to the present teachings may include one or more features of such a conventional swab of the prior art-e.g., they may be made from the same or similar materials, and/or include the same or similar sizes/dimensions. For example, the swabs according to the present teachings may include similar lengths, widths, diameters, surface areas, shapes, and so on, relative to a conventional swab of the prior art (e.g., the length and/or diameter of the swab as a whole, the length and/or diameter of the shaft, the length and/or diameter of the collection tip, and so on, may be the same or similar to conventional swabs of the prior art). Also, or instead, it will be understood that swabs according to the present teachings may be manufactured in the same or a similar manner relative to conventional swabs of the prior art.

Fig. 2 illustrates a compound swab in a coupled state, in accordance with a representative embodiment, and Fig. 3 illustrates a compound swab in a decoupled state, in accordance with a representative embodiment. This compound swab or "twin swab" may include two or more swabs that can be separated after sample collection, where one swab can contribute to the pool for pool testing, and the other can be set aside for testing based on the results of the pool testing (e.g., if the pool tests positive). The swab of Fig. 2 may represent a compound swab after collection but before separation, where the swabs that form the compound swab are coupled together through mechanical means, a magnetic force, an adhesive, combinations thereof, or the like.

It will be understood that such a compound swab (or "twin-swab") according to aspects of the present teachings can have many advantages for specimen collection, particularly in pool testing applications. For example, a compound swab as described herein can increase the efficiency of pool testing by reducing the amount of testing required (which can be particularly advantageous when performing reverse transcriptase polymerase chain reaction (RT-PCR) tests, e.g., by unburdening laboratories performing such tests and conserving materials such as reagent chemicals that may be required for such tests), reducing the number of swabs utilized, and reducing the number of swabbing per test. In this manner, a compound swab as described herein can enable effective and efficient pool testing, thereby increasing testing capabilities, which can aid in safely and effectively managing a pandemic such as the global pandemic in 2020 due to COVID-19. By way of example, a compound swab as described herein can aid in testing on a mass scale-e.g., in mass testing sites, schools, sports leagues, sporting events (or other events and gatherings), businesses and offices, entertainment venues, manufacturing facilities, warehouses, prisons/jails, healthcare facilities, long-term care facilities, and the like.

The swab shown in Figs. 2 and 3 may generally include a device 200 for collecting a specimen (e.g., a biological specimen) for analysis and/or testing. The device 200 may include a plurality of swabs-e.g., a first swab 210 (labeled "Swab 1" in the figure) including a first shaft 212 and a first collection tip 214 disposed on an end of the first shaft 212, where the first collection tip 214 is structurally configured to acquire a first portion of a specimen thereon (thus the labeling of the first collection tip 214 as "Specimen" in the figure); and a second swab 220 (labeled "Swab 2" in the figure) including a second shaft 222 and a second collection tip 224 disposed on an end of the second shaft 222, where the second collection tip 224 is structurally configured to acquire a second portion of the specimen thereon (thus the labeling of the second collection tip 224 as "Specimen" in the figure). The device 200 may generally be structurally configured for collecting a specimen (e.g., during one sample collection operation) from an animal, where the specimen from the animal accumulates on both the first collection tip 214 and the second collection tip 224.

As shown in Fig. 3, the device 200 may further include one or more first couplers 331 disposed along the first swab 210, and one or more second couplers 332 disposed along the second swab 220. The first couplers 331 may be removably engageable with the second couplers 332 to establish a compound swab to collect the specimen on each of the first collection tip 214 and the second collection tip 224, where disengagement of the first couplers 331 and the second couplers 332 permits separate testing of the specimen by separating the different swabs of the compound swab. In this manner, the first swab 210 may be used in a pool testing procedure, and the second swab 220 may be preserved for testing of the specimen based on the results of the pool testing procedure, or vice-versa.

As shown in the figures, one or more of the first couplers 331 and one or more of the second couplers 332 may include at least one of a projection and a void. That is, the first couplers 331 may include projections and the second couplers 332 may include voids, or vice-versa or combinations thereof (e.g., the first couplers 331 may include both projections and voids, and the second couplers 332 may include corresponding voids and projections). Also or instead, one or more of the first couplers 331 and one or more of the second couplers 332 may include a hook and loop fastener. Other mechanical arrangements are also or instead possible for one or more of the first couplers 331 and one or more of the second couplers 332 for coupling and decoupling the swabs including but not limited to use of one or more of a clamp, a clip, a dowel, a friction fit, an adhesive, a latch, a lock, a pin, a screw, a bolt, a snap fit, a magnet, a button, a zipper, interlocking teeth, another fastener, and so on. Further, one or more of the first couplers 331 and second couplers 332 for coupling and decoupling the swabs may be permanently or temporarily affixed on the device 200. For example, one or more of the first couplers 331 and second couplers 332 may be configured to be broken off of one or more of the first swab 210 and the second swab 220 during a decoupling operation. Also or instead, one or more of the first couplers 331 and second couplers 332 may be structurally configured to have at least a portion thereof remain engaged with one or more of the first swab 210 and the second swab 220 after decoupling of the swabs. For example, where one or more of the first couplers 331 and second couplers 332 is disposed on or near one or more of the first collection tip 214 and the second collection tip 224, it may be advantageous that such couplers are not completely disengaged from the first swab 210 and the second swab 220 after decoupling because such couplers could contain a biological specimen thereon that can contaminate a surrounding environment, lab personnel, and the like.

The couplers may be included on the shaft of the swab, the collection tip of the swab, or both. For example, one or more of the first couplers 331 may be disposed on the first shaft 212 and one or more of the second couplers 332 may be disposed on the second shaft 222. Also or instead, at least one of the first couplers 331 may be disposed on the first collection tip 214, and at least one of the second couplers 332 may be disposed on the second collection tip 224.

In an aspect, the first collection tip 214 and the second collection tip 224 are made of one or more of polyester, rayon, and flocked nylon. By way of example, a manufacturing process for the device 200 may include forming the main structure of the device 200 (e.g., the shaft, which can be formed via injection molding, die cutting, extrusion, casting, and so on), and then adding the collection material to one or more tips thereof thereby forming the collection tip(s). To this end, the shape of the main structure of the device 200 near the collection tips may be structurally configured to promote a predetermined shape after a collection material is added thereon. This may include, for example, a substantially uniform shape, a taper, a flared or bulbous end, and the like. It will be understood that, in embodiments where one or more couplers are disposed on or near the collection tip(s), the couplers may be formed on a structure of the collection tip(s) prior to application of the collection material being added during manufacturing (e.g., by flocking or the like). Alternatively, one or more couplers may be formed on a collection tip after or during application of the collection material.

In an aspect, the first shaft 212 and the second shaft 222 are made of one or more of a plastic material, a wood, and a metal. Other materials are possible. Regardless, in an aspect, the shafts may be substantially flexible. That is, in an aspect, the first shaft 212 and the second shaft 222 are selectively flexible (e.g., via movement by a user through anatomy of an animal such as a nasal passage of a human) to conform to at least a portion of a cavity, e.g., a nasal passage of an animal. In this manner, the specimen may be a nasopharyngeal specimen, and the device 200 may be structurally configured for collection of the nasopharyngeal specimen from a surface of respiratory mucosa of an animal.

In an aspect, at least a portion of one or more of the first collection tip 214 and the second collection tip 224 may be removable from its corresponding shaft. This may be facilitated by material selection of the collection tips, by serrating the collection tips or otherwise creating a relatively weak connection point in the collection tips, combinations thereof, or similar.

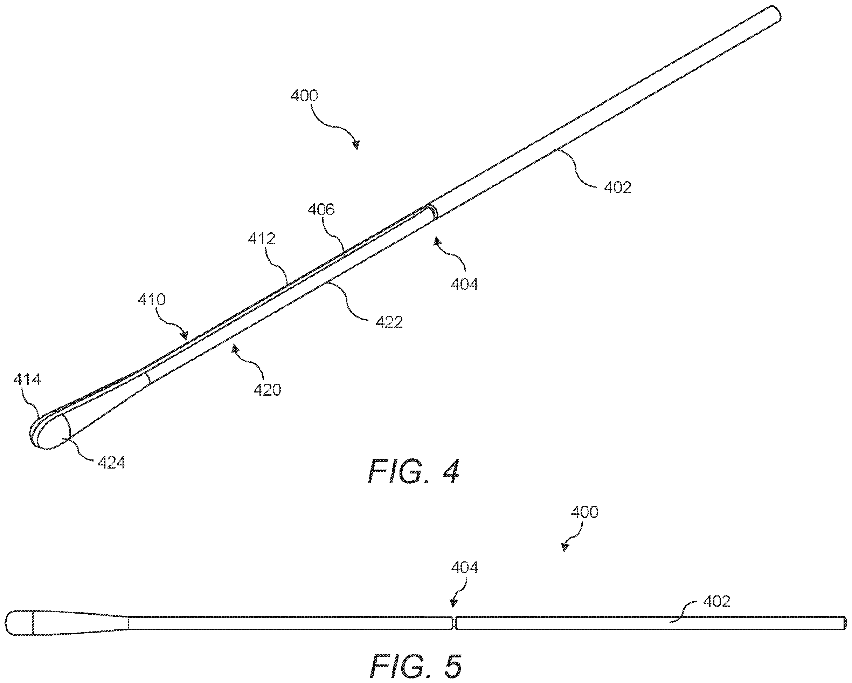

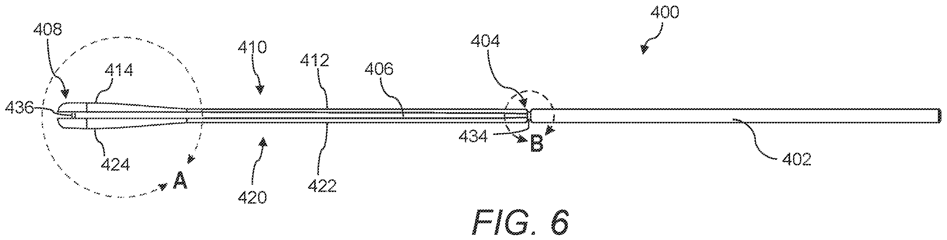

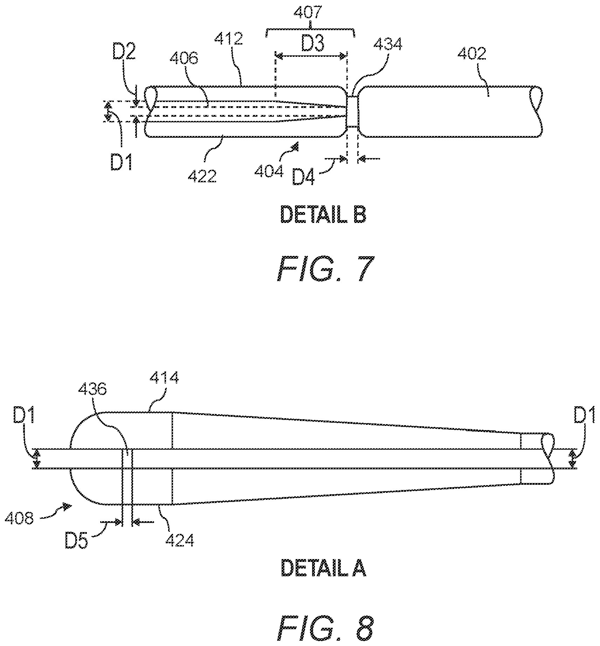

Turning to Figs. 4-8, an aspect of the present teachings may include a device 400 where a plurality of swabs are attached (in a coupled state) to a common shaft 402 at a first breakpoint 404 for separating the swabs. Specifically, Fig. 4 illustrates a compound swab in accordance with a representative embodiment, Fig. 5 illustrates a top view of the compound swab of Fig. 4, Fig. 6 illustrates a side view of the compound swab of Fig. 4, Fig. 7 illustrates `Detail B' of Fig. 6, and Fig. 8 illustrates `Detail A' of Fig. 6. It will be understood that the embodiment of the compound swab shown in Fig. 4 may be similar to the embodiments described above or elsewhere herein, and may thus include any of the features described with reference to those other embodiments, and vice-versa.

As shown in Fig. 4, the compound swab device 400 may include a first swab 410 including a first shaft 412 and a first collection tip 414 disposed on an end of the first shaft 412, where the first collection tip 414 is structurally configured to acquire a first portion of a specimen thereon; and a second swab 420 including a second shaft 422 and a second collection tip 424 disposed on an end of the second shaft 422, where the second collection tip 424 is structurally configured to acquire a second portion of the specimen thereon. That is, each of the two (or more) collection tips of the compound swab device 400 may be structurally configured to acquire a portion of a specimen thereon during the same specimen collection procedure. In the compound swab device 400 of Fig. 4, the first swab 410 and the second swab 420 may be releasably connected to a common shaft 402 at a first breakpoint 404. More particularly, the first shaft 412 and the second shaft 422 may be coupled to the common shaft 402, directly or indirectly. Above the coupling to the common shaft 402, the first swab 410 and the second swab 420 may be disposed adjacent to one another, but separated from one another for all of, or a substantial portion of, their lengths. For example, the first swab 410 and the second swab 420 may be disposed substantially parallel to one another and separated by a substantially fixed distance (or a variable distance) from one another when coupled via the common shaft 402 and/or other connectors. This separation may form a channel 406 or other void between the first swab 410 and the second swab 420, where this channel 406 can aid in separation of the first swab 410 and the second swab 420 from each other and the common shaft 402. For example, the channel 406 can provide a gripping area for orienting a user when pulling apart the swabs.

As shown in Fig. 6, the first swab 410 and the second swab 420 may be connected-at least temporarily, in the coupled state of the compound swab device 400-at one or more locations. These connected locations may include one or more breakpoints, where the coupling between the first swab 410 and the second swab 420 can be broken and/or disengaged after sample collection to separate/decouple the swabs for pool testing or the like as described herein. In some implementations, and as shown in Fig. 6, a compound swab device 400 may include a plurality of such breakpoints. For example, the compound swab device 400 may include at least a first breakpoint 404 at or near the common shaft 402, and a second breakpoint 408 along the length of the first swab 410 and the second swab 420. One or more of these breakpoints may include a coupler as described herein-for example, the first breakpoint 404 may include a first coupler 434 engaged to each of the first swab 410 and the second swab 420 in a coupled state, and the second breakpoint 408 may include a second coupler 436 engaged to each of the first swab 410 and the second swab 420 in a coupled state. Figs. 7 and 8 include close-up views of each of these example breakpoints for the compound swab device 400 of Fig. 6.

Before describing Figs. 7 and 8, example dimensions for the compound swab device 400 are provided. It will be understood, however, that alternative dimensions can be used, and in fact should be expected, depending upon a variety of factors including without limitation the materials used for the device 400, applications for the device 400, and so on. Thus, these dimensions, and any dimensions presented herein, will be understood to be provided by way of example and not limitation. To this end, an example embodiment of the compound swab device 400 may have an overall length of about 149.94 millimeters, the collection tips may have a length of about 21.94 millimeters, the common shaft 402 may have a length of about 69.75 millimeters, and the shafts of each swab (the first shaft 412 and the second shaft 422) may have lengths of about 57.75 millimeters, where the remaining 0.50 millimeters defines a junction or collar between the common shaft 402 and the shafts of each swab.

As shown in Fig. 7, which generally shows a detail (i.e., Detail B from Fig. 6) of the first breakpoint 404 for the first swab 410 and the second swab 420, and more specifically for the first shaft 412 and the second shaft 422 to be detached from each other and the common shaft 402. As shown in the figure, the first coupler 434 may include a collar that each of the first shaft 412 and the second shaft 422 are connected to when the first swab 410 and the second swab 420 are in a coupled state. The first coupler 434 (e.g., when formed as a collar as shown in the figure, or otherwise) may be disposed between the common shaft 402 and the swabs where the first coupler 434 is coupled to each of the foregoing. Also or instead, the first coupler 434 may be an extension of the common shaft 402 (e.g., it may be integral with the common shaft 402), an extension of one or more of the swabs (e.g., it may be integral with one or more of the first shaft 412 and the second shaft 422), and/or it may be a separate element from one or more of the common shaft 402, the first swab 410, and the second swab 420.

As also shown in Fig. 7, the channel 406 may include a tapered portion 407 or another similar feature at or near one or more of the breakpoints. For example, as shown in the figure, the channel 406 may generally define a first distance D1 throughout a substantial portion of its length between the first swab 410 and the second swab 420, where the tapered portion 407 yields a smaller distance than D1-i.e., dimension D2 as shown in the figure-between the first swab 410 and the second swab 420. The tapered portion 407 may occur over a certain length D3 of the channel 406. By way of example and not of limitation, in an aspect, D1 is about 1.00 millimeter, D2 is about 0.44 millimeters, and D3 is about 3.50 millimeters, where the width of a collar that forms the first coupler 434 is about 0.50 millimeters. It will be understood that alternative dimensions (and shapes) are also or instead possible.

The tapered portion 407 or another similar feature may be provided for enhanced stability of the overall device 400 when in the coupled state. That is, the relatively narrow shafts of the swabs-the first shaft 412 and the second shaft 422-may benefit from the inclusion of a connection to the common shaft 402 that is thicker than the dimension that makes up a majority of the length of the relatively narrow shafts (i.e., a thicker base portion). More specifically, in certain aspects, the increased stability provided by the tapered portion 407 or another similar feature may allow for a collection material to be added on a tip of the shafts to form the collection tips of the device 400 during a manufacturing process. Thus, certain aspects of the device 400 that include a tapered portion 407 or another similar feature may have relatively larger channels 406 throughout a majority of the length of the shafts than aspects that do not include such tapers.

Fig. 8 generally illustrates a detail (i.e., Detail A from Fig. 6) of the second breakpoint 408 for the first swab 410 and the second swab 420, and more specifically in some aspects, for the first collection tip 414 and the second collection tip 424 to be detached from each other. As shown in the figure, the second coupler 436 may include a linkage that connects at least a portion of the first swab 410 to at least a portion of the second swab 420-e.g., where the linkage connects the first collection tip 414 and the second collection tip 424-when the first swab 410 and the second swab 420 are in a coupled state. The second coupler 436 may be an extension of one or more of the swabs (e.g., it may be integral with one or more of the first collection tip 414 and the second collection tip 424), and/or it may be a separate element from one or more of the first swab 410 and the second swab 420. When the swabs are decoupled, the second coupler 436 or a portion thereof may remain connected to one or both of the first swab 410 and the second swab 420. Alternatively, the second coupler 436 may be completely detached from one or both of the first swab 410 and the second swab 420 when the swabs are decoupled. In an implementation, it may be preferable that the entire second coupler 436 remain connected to one or more of the first swab 410 and the second swab 420 after decoupling the swabs, particularly when disposed near the collection tips thereof, to avoid the second coupler 436- which may contain a portion of a biological specimen thereon-from contaminating an environment and/or personnel.

In an implementation, the distance between the first collection tip 414 and the second collection tip 424 may be equal to the first distance D1 described above, which may be about 1.00 millimeters. This distance may or may not be uniform in certain aspects. And, in an implementation, the thickness of the second coupler 436, dimension D5 in the figure, may be about 0.50 millimeters.

In use, and after a sample collection procedure, the first swab 410 and the second swab 420 may be separated (or partially separated) from one another, and/or decoupled from the common shaft 402, via a force applied to the device 400. Specifically, the force may include a user pulling the first swab 410 and the second swab 420 apart, which may break the connection formed by one or more couplers included thereon, e.g., the first coupler 434 and the second coupler 436. Also or instead, the force may include a user twisting or otherwise providing a torque to the device 400, which may break the connection formed by one or more couplers included thereon, e.g., the first coupler 434 and the second coupler 436. Other techniques for decoupling, including other forces applied to the device 400, are also or instead possible.

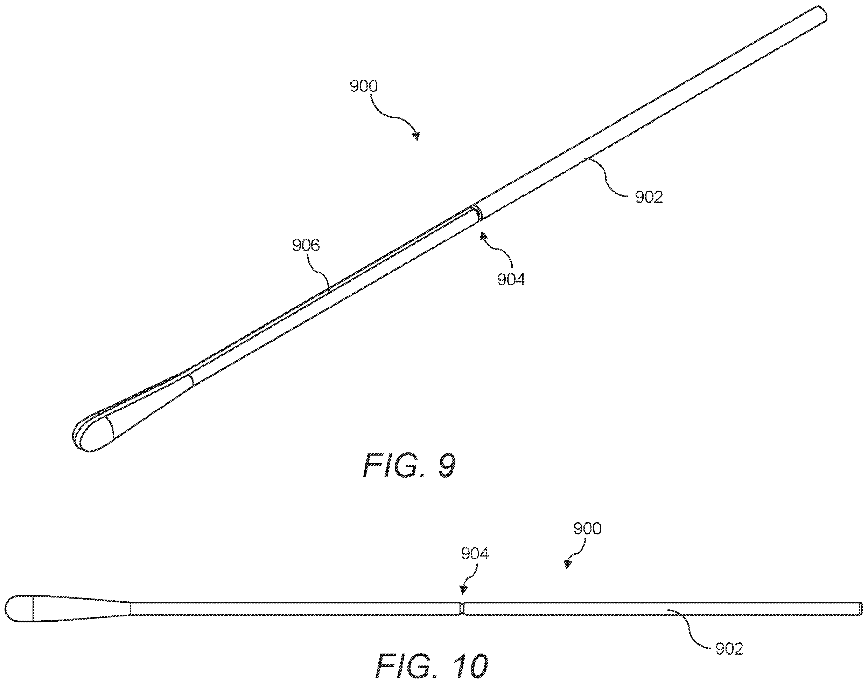

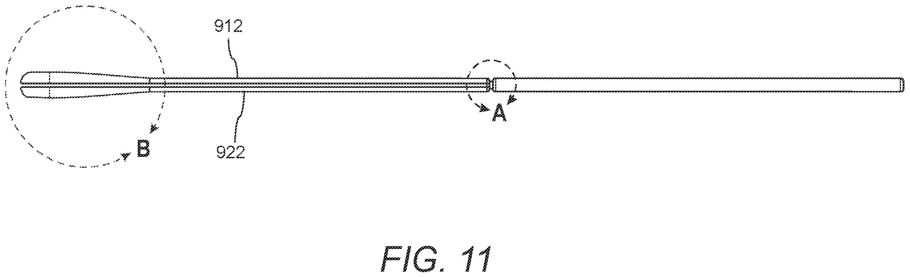

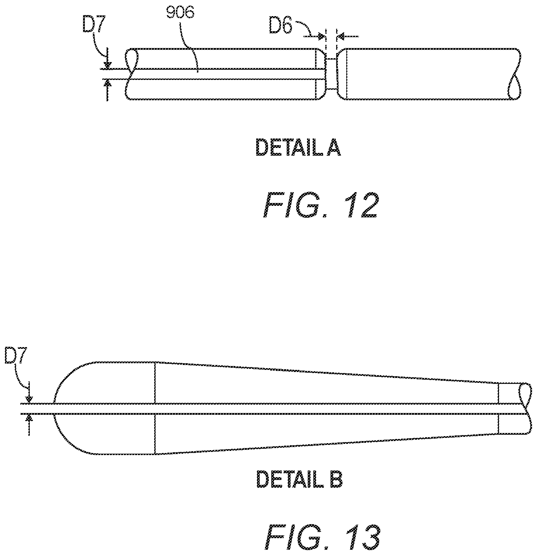

Turning to Figs. 9-13, an aspect of the present teachings may include a device 900 that is similar to that described above for Figs. 4-8, i.e., where a plurality of swabs are attached (in a coupled state) to a common shaft 902 at a first breakpoint 904 for separating the swabs. However, in this embodiment, the first breakpoint 904 may be the only breakpoint for the device 900. In any event, Fig. 9 illustrates a compound swab in accordance with a representative embodiment, Fig. 10 illustrates a top view of the compound swab of Fig. 9, Fig. 11 illustrates a side view of the compound swab of Fig. 9, Fig. 12 illustrates 'Detail A' of Fig. 11, and Fig. 13 illustrates `Detail B' of Fig. 11. It will be understood that the embodiment of the compound swab shown in Fig. 9 may be similar to the embodiments described above or elsewhere herein, and may thus include any of the features described with reference to those other embodiments, and vice-versa.

The overall dimensions of the compound swab device 900 may be the same as, or similar to, the dimensions of the device 400 described above. Thus, an example embodiment of the compound swab device 900 may have an overall length of about 149.99 millimeters, the collection tips may have a length of about 21.99 millimeters, the common shaft 902 may have a length of about 69.75 millimeters, and the shafts of each swab (the first shaft 912 and the second shaft 922) may have lengths of about 57.58 millimeters, where the remaining 0.50 millimeters defines a junction or collar between the common shaft 902 and the shafts of each swab (see dimension D6 in Fig. 12, where D6 may be about 0.50 millimeters). However, because the first breakpoint 904 may be the only breakpoint for the device 900, the channel 906 may be narrower than that described above. Thus, in an implementation, dimension D7 in Figs. 12 and 13 may only be about 0.50 millimeters. Moreover, in the embodiment shown in Figs. 9-13, a tapered portion or other similar feature may not be needed or desired for stability because the channel 906 may be narrower and more stable without such a feature.

It will be understood that, although two breakpoints are described with respect to Figs. 4-8 and one breakpoint is described with respect to Figs. 9-13, implementations may include more breakpoints.

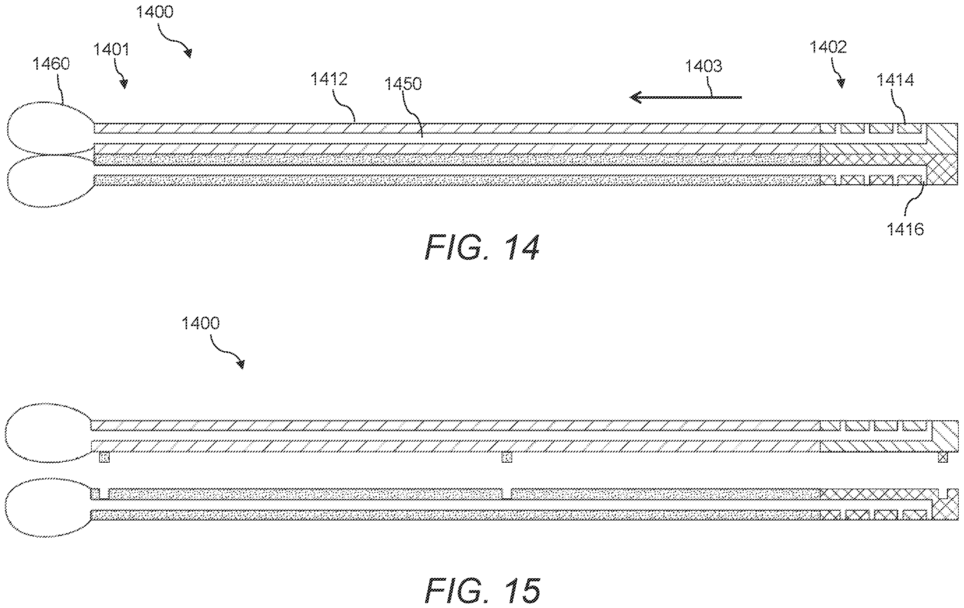

Fig. 14 illustrates a cross-section of a swab for collecting a specimen for testing, in accordance with a representative embodiment, and Fig. 15 illustrates a cross-section of a swab for collecting a specimen for testing, in accordance with a representative embodiment. Specifically, the swabs 1400 shown in these figures may be a compound swab as described herein, e.g., multiple swabs separable for independent analysis thereof after collective sample retrieval, which can be useful for pool testing and the like. Thus, it will be understood that the swab 1400 of Figs. 14-15 may include any of the features of the other swabs described herein. The swab 1400 may also include a feature (e.g., an air "pillow") that allows a user to create suction at a collection end of the swab 1400-e.g., by pushing air through small channels in the swab parts. Upon release of such an air pillow during the sampling process, the negative pressure exerted by the re-expanding air pillow may create a force that facilitates the aspiration of samples and by doing so aids in the sample recovery. Of note, the suction-enhanced sample collection of the present teachings can also or instead be used with conventional, single swabs.

In general, the swab 1400 may be structurally configured for collecting a specimen for testing and/or analysis. The swab 1400 may include a shaft 1412 having a first end 1401 and a second end 1402, where the shaft 1412 includes a channel 1450 disposed therethrough from the second end 1402 toward the first end 1401. For example, the channel 1450 may extend completely through the shaft 1412 as shown, or partially through the shaft 1412 (e.g., midway, or otherwise toward the first end 1401).

The swab 1400 may also include a collection tip 1414 disposed on the second end 1402 of the shaft 1412, where the collection tip 1414 is structurally configured for acquiring a specimen thereon. The collection tip 1414 may be permeable to permit a flow of fluid (e.g., air) therethrough. For example, the collection tip 1414 may include one or more suction features 1416 such as one or more of vents, holes, sub-channels, and the like that establish fluid communication between an external environment and the channel 1450. Also or instead, a material of the collection tip 1414 may provide for the permeability of the collection tip 1414 and may establish fluid communication between an external environment and the channel 1450.

The swab 1400 may further include a suction enhancer 1460 in fluid communication with the channel 1450. The suction enhancer 1460 may be activatable to establish a flow of fluid through the channel 1450, e.g., in a first direction 1403 from the second end 1402 toward the first end 1401. This flow of fluid may thereby establish suction at the second end 1402 of the shaft 1412 through the collection tip 1414 (e.g., through the suction features 1416). While there are many devices that can create the flow of fluid through the channel 1450 and thus could be used as a suction enhancer 1460 of the present teachings, preferred embodiments may be relatively simple for advantageous manufacturing, cost, and/or ease of use. By way of example, the suction enhancer 1460 may include a fluid reservoir compressible to a first state, where, when the fluid reservoir is released from the first state, the fluid reservoir draws in fluid from the channel 1450 thereby establishing the flow of fluid in the first direction 1403. Such a fluid reservoir may include a bag, a cushion, a pillow, or the like, e.g., where the first state represents a state where a user squeezes the fluid reservoir to compress it into the first state, and where a user merely releases the compression of the fluid reservoir to draw air therein thereby providing suction. This suction may be configured to promote capture of a specimen on the collection tip 1414.

Thus, the present teachings may include a suction-enhanced compound swab, where, prior to collection of a specimen, air is forced out from a fluid reservoir (e.g., of a suction enhancer 1460) through compression thereof by a user (e.g., squeezing with their fingers), and where, upon release of such compression of the fluid reservoir, air and specimen are sucked within and onto, respectively, the swab 1400.

Similar to other compound swabs described herein, the swab 1400 may further include a second shaft removably coupled to the shaft 1412, the second shaft having a first end and a second end, and the second shaft including a second channel disposed therethrough. The swab 1400 may thus also include a second collection tip disposed on the second end of the second shaft and structurally configured for acquiring the specimen thereon, where the second collection tip is permeable to permit a flow of fluid therethrough. It will be understood that such a compound swab may include one or more suction enhancers 1460-e.g., a suction enhancer 1460 for each of the swabs, or one suction enhancer 1460 for collective use by all (or a plurality) of the swabs. Thus, in an aspect, the suction enhancer 1460 is in fluid communication with the second channel (e.g., the same suction enhancer 1460 that is also in fluid communication with the channel 1450 of another swab). In this manner, the channel 1450 and a second channel of another swab may be in fluid communication with one another. Instead, and as shown in the figure, the swab 1400 may include a second suction enhancer in fluid communication with the second channel, where the channel 1450 and the second channel are not in fluid communication with one another.

The swab 1400 may be used in a method for collecting a specimen for testing, where the method includes inserting a swab within an environment to collect a specimen (e.g., inserting a swab within an orifice of an animal to collect a biological specimen from the animal). The swab may include a shaft having a first end and a second end, the shaft including a channel disposed therethrough from the second end toward the first end; and a collection tip disposed on the second end of the shaft and structurally configured to acquire a specimen thereon, where the collection tip is permeable to permit a flow of fluid therethrough. The method may also include activating a suction enhancer in fluid communication with the channel, establishing a flow of fluid through the channel in a first direction from the second end toward the first end, and establishing suction at the second end of the shaft through the collection tip to promote collection of the specimen. As discussed herein, the suction enhancer may include a fluid reservoir, where activating the suction enhancer includes releasing the fluid reservoir from a first state in which the fluid reservoir is compressed. In this manner, the method may further include, before insertion of the swab within the environment (e.g., before insertion of the swab within the orifice of the animal), compressing the fluid reservoir thereby placing the fluid reservoir in the first state. It is also understood, however, that compression of the fluid reservoir can occur when the collection tip of the swab is inserted within the environment for collection, e.g., in circumstances where a flow or "puff' of air can be beneficial to promote capture of a specimen.

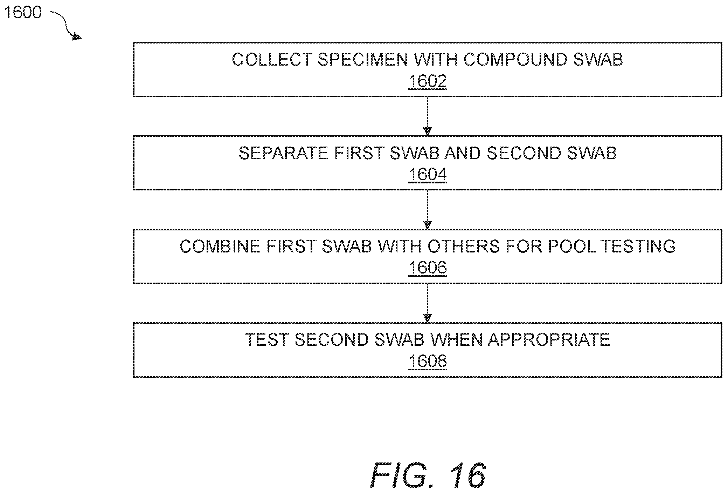

Fig. 16 is a flow chart of a method of pool testing, in accordance with a representative embodiment. Specifically, the method 1600 may be implemented using one or more of the swabs disclosed herein according to aspects of the present teachings.

As shown in step 1602, the method 1600 may include collecting a specimen using a compound swab. The compound swab may be any one of the swabs disclosed herein. For example, the compound swab may include a first swab having a first shaft and a first collection tip disposed on an end of the first shaft, and a second swab coupled to the first swab, where the second swab includes a second shaft and a second collection tip disposed on an end of the second shaft. Collecting a specimen using a compound swab may be accomplished by inserting the compound swab within an orifice of an animal to collect a biological specimen from the animal. The method 1600 may thus also include removing the compound swab from collection of the specimen (e.g., removing the compound swab from the orifice of an animal with portions of the biological specimen disposed on each of the first collection tip and the second collection tip).

As shown in step 1604, the method 1600 may include separating the first swab and the second swab. And, as shown in step 1606, the method 1600 may include combining the first swab with a plurality of different swabs for pool testing the specimen with other, different specimens for the presence or absence of a component of interest (e.g., organisms or other clinical markers for disease, and so on). The second swab may be set aside for later use, if necessary or desired. Thus, the method 1600 may further include labeling and/or storing the second swab.

As shown in step 1608, the method 1600 may include testing the second swab for the presence or absence of the component, when appropriate or desired. That is, in certain implementations, based on a result of the pool testing, the method 1600 may include selectively testing the second swab. For example, if the pool testing comes back showing that the pool tested positive for a particular characteristic of interest (e.g., the presence of organisms or other clinical markers for disease), it may be desirous to know which specimen(s) within the pool triggered this positive test result. Thus, the second swab may be tested to see if the subject associated with that particular compound swab was one of the subjects (or the only subject) that caused the positive pool test result. Conversely, if the pool testing is negative (e.g., showing that, within the pool, there is/are none of the characteristic(s) of interest such as the presence of organisms or other clinical markers for disease), then the second swab (and other corresponding portions of compound swabs related to first swabs tested in the pool testing) may be discarded and/or simply not subjected to further testing. In this manner, the method 1600 may be used to conserve resources such as lab supplies, lab personnel, time, and so on.

Devices, systems, and methods for storing, transporting, and/or facilitating the collection of samples for pool testing and the like will now be described. It will be understood that these devices, systems, kits, and techniques may be used with any of the swabs as disclosed herein, e.g., a compound swab.

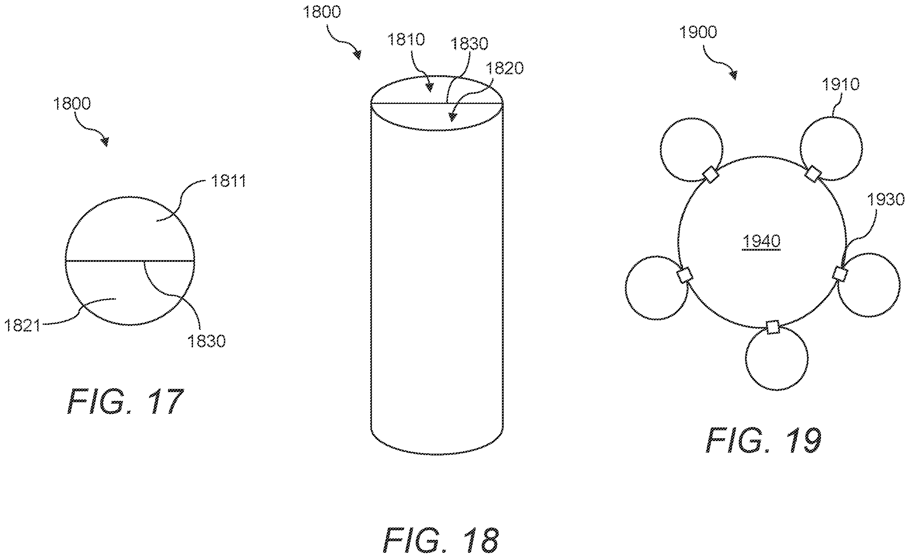

Fig. 17 illustrates a top view of a container for receiving a swab, in accordance with a representative embodiment, and Fig. 18 illustrates a container for receiving a swab, in accordance with a representative embodiment. The container 1800 shown in these figures may be part of a system for collecting a specimen for testing that includes a compound swab according to the present teachings-e.g., a compound swab including a first swab having a first shaft and a first collection tip disposed on an end of the first shaft, and a second swab removably coupled to the first swab, where the second swab includes a second shaft and a second collection tip disposed on an end of the second shaft. That is, the container 1800 may be structurally configured to separate and/or house one or more of the swabs of a compound swab.

Therefore, in general, the container 1800 may include one or more collection regions (e.g., tubes) or the like. And using this container 1800, a compound swab can be separated in one or more collection regions by a separator 1830 such as a relatively sharp obstacle or similar (e.g., an edge, which may include a taper). After separation, one of the swabs can remain in a collection region for later individual testing (e.g., if necessary or desired), and another one of the swabs can be collected in another region (e.g., tube or the like) with other swabs for pool testing.

The container 1800 may thus be structurally configured to receive one or more swabs therein, e.g., one or more of a first swab and a second swab of a twin swab. The container 1800 may include a first opening 1811 leading to a first chamber 1810, where the first chamber 1810 is structurally configured to contain at least a portion of one or more swabs therein.

The separator 1830 may be disposed within or adjacent to one or more of the first opening 1811 and the first chamber 1810. The separator 1830 may be sized and shaped to fit between at least a first swab and a second swab of a compound swab, e.g., wedged between the collection tips and/or shafts thereof. In this manner, when the separator 1830 is disposed at least partially between the swabs, a predetermined force placed on the compound swab that is directed toward the container 1800 may separate the swabs. The separator 1830 may be located on the container 1800 such that, upon separation of swabs using the separator 1830, one or more of the swabs will be at least partially disposed within the first chamber 1810. The other swab(s), upon separation, may be disposed within another chamber of the container 1800 or the other swab(s) may be located external to the container 1800 (e.g., in an aspect where the separator 1830 is disposed on an upper or outer edge of the container 1800). The separator 1830 may also or instead be disposed within the container 1800, e.g., toward a top of the container 1800, in a middle section of the container, toward a bottom of the container 1800, or elsewhere.

In certain aspects, the container 1800 may include a second opening 1821 leading to a second chamber 1820. The separator 1830 may be disposed between a boundary of the first opening 1811 and the second opening 1821, and in some implementations, the separator 1830 may form a boundary between chambers of the container 1800.

The container 1800 may be used in a method for storing a specimen, where the method includes aligning a separator 1830 between a first swab and second swab of a compound swab, where the separator 1830 is coupled to a container 1800 including a first opening 1811 leading to a first chamber 1810, and where the first chamber 1810 is structurally configured to contain at least a portion of one or more of the first swab and the second swab therein. The method may further include applying a predetermined force against the separator 1830 and toward the container 1800 with the compound swab thereby separating the first swab and the second swab, where at least the portion of one or more of the first swab and the second swab is disposed within the first chamber 1810 upon separation thereof.

Fig. 19 illustrates a top view of a container for receiving a swab, in accordance with a representative embodiment. The container 1900 shown in this figure includes a plurality of individual swab containers 1910 and separators 1930, and one pool container 1940. In this manner, compound swabs can be separated where one swab is disposed within the individual swab container 1910 upon separation, and one swab is disposed within the pool container 1940 (e.g., with other swabs) upon separation. The individual swab containers 1910 may be removable from the pool container 1940.

It will be understood that one or more of the containers and/or receptacles described herein may include covers or caps (e.g., screw caps) to protect specimens from external environments.

An aspect of the present teachings may include a receptacle configured to collect multiple swabs and multiple specimens, e.g., for pool testing. In general, the receptable may feature a "push-twist-pull" receptable-e.g., where a user (i) pushes a swab into the receptacle, (ii) twists the receptable to break off a portion of the collection tip that then collects in a predefined portion of the receptable, and (iii) pulls the predefined portion off of the receptable for transport for analysis/testing (e.g., pool testing). The receptable may generally include at least two parts-an upper part that serves to collect swabs in an organized manner and stabilizes them mechanically for the next step, which can include the severing of a part of the specimen. The lower part of the receptacle may collect the severed specimen piece(s). To this end, this part of the receptacle can be prefilled with a transport medium or the like, if desired. The chopping/severing process may include the application of a rotational force or the like by a user. Release and enrichment of a specimen can be achieved by biochemical or chemical modification of the conventional transport medium or receptacle, e.g., through the use of additives, material, and/or immobilized binding partners with high affinity to the target. Once the severing has occurred, the specimen may drop into the lower receptacle, where the collection of a plurality of specimens can be accommodated. In this manner, a swab with the partially chopped-off sample may be set aside for testing, e.g., when the pool tests positive.

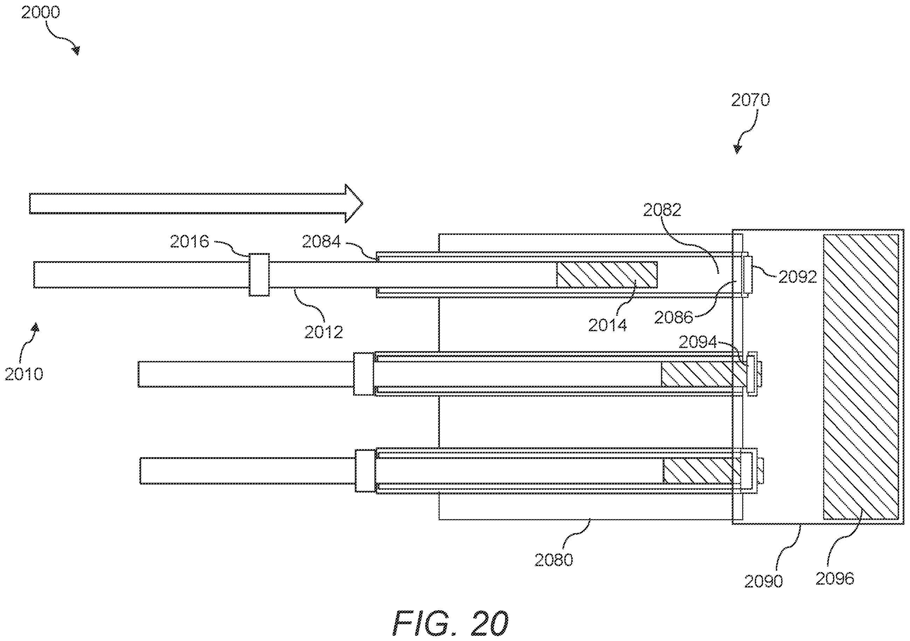

Fig. 20 illustrates a cross-section of a receptacle for collection of a plurality of specimens, in accordance with a representative embodiment. More particularly, this figure shows a kit 2000 or system featuring a plurality of swabs 2010, where each of the plurality of swabs 2010 includes a shaft 2012 and a collection tip 2014 disposed on an end of the shaft 2012. The kit 2000 may further include a receptacle 2070 structurally configured for the collection of a plurality of specimens. The receptacle 2070 may generally include a first housing 2080 and a second housing 2090, where these housings may be movably coupled to one another and/or removable from one another.

The first housing 2080 of the receptacle 2070 may include a plurality of first bores 2082 extending through the first housing 2080 from a top end to a bottom end. Each of the plurality of first bores 2082 may include a top opening 2084 configured to receive a swab 2010 therein and a bottom opening 2086 configured to project at least a portion of a collection tip 2014 of the swab 2010 therefrom.

The second housing 2090 of the receptacle 2070 may be couplable to the first housing 2090. The second housing 2090 may be movable relative to the first housing 2080 between a first position and a second position. The second housing 2090 may include a plurality of second bores 2092, e.g., where one or more of the number, size, and shape of the second bores 2092 may correspond to one or more of the number, size, and shape of the first bores 2082. In this manner, each of the plurality of second bores 2092 may include a collection opening 2094 that is aligned with the bottom opening 2086 of a first bore 2082 and that is configured to receive at least a portion of the collection tip 2014 of the swab 2010 therein when the second housing 2090 is in the first position. Thus, the bores of the receptacle 2070 may be aligned when it is in the first position. Movement of the second housing 2090 from the first position to the second position may remove alignment of the collection opening 2094 with the bottom opening 2086 of the first bore 2082. Further, this movement of the second housing 2090 from the first position to the second position may sever or otherwise disconnect at least a portion of the collection tip 2014 from the swab 2010. That is, when at least a portion of the collection tip 2014 of the swab 2010 is disposed within one of the plurality of second bores 2092 and the second housing 2090 is moved from the first position to the second position, an abutment between the first housing 2080 and the second housing 2090 may be structurally configured to sever at least a portion of the collection tip 2014 from the swab 2010. For example, the receptacle 2070 may include a cutting edge or the like disposed at the abutment between the first housing 2080 and the second housing 2090. Also, or instead, the swabs 2010 may include collection tips 2014 that are predisposed to having at least a portion broken off, e.g., through the inclusion of a serration or the like.

The receptacle 2070 may further include a collection volume 2096 within the second housing 2090 that is structurally configured to receive at least a portion of the collection tip 2014 that is severed from the swab 2010. In this manner, in an aspect where the second housing 2090 is removable from the first housing 2080, the collection volume 2096 can serve as a transport medium for one or more specimens to be tested. The collection volume 2096 may thus include (e.g., physically contain) a transport medium or the like.

Movement from the first position to the second position may include rotation of the second housing 2090 relative to the first housing 2080. Other movement is also or instead possible. Further, the receptacle 2070 may include one or more mechanical stops to limit movement of the second housing 2090 relative to the first housing 2080.

In the kit 2000, one or more of the swabs 2010 may be preconfigured for use with the receptacle 2070. For example, there may be a limiter 2016 disposed on one or more of the plurality of swabs 2010, the first housing 2080, and the second housing 2090. Such a limiter 2016 may be structurally configured to limit projection of the portion of the collection tip 2014 from the bottom opening 2086 of the first bore 2082 to a predetermined amount. By way of example, and as shown in the figure, the limiter 2016 may include a projection or the like on the shaft 2012 of one or more of the plurality of swabs 2010. Other forms of mechanical stops, mating features, and/or limiters are also or instead possible.

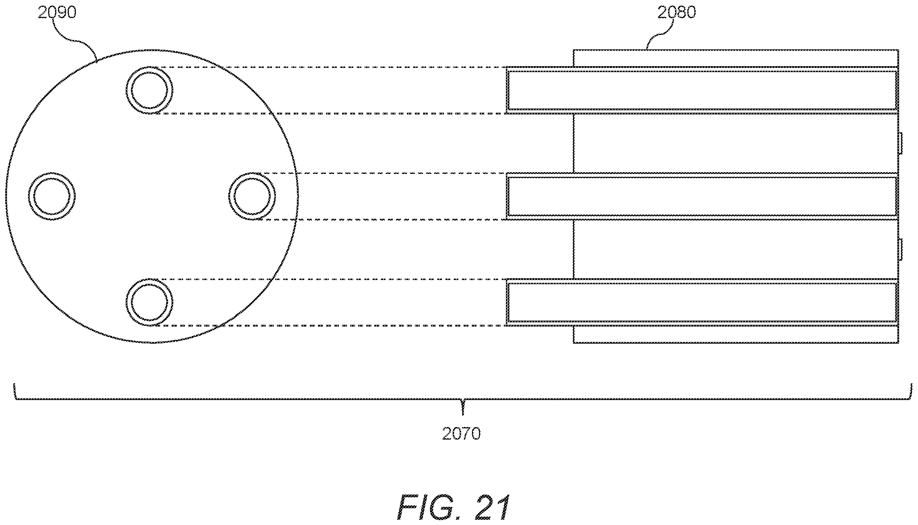

Fig. 21 illustrates a receptacle for collection of a plurality of specimens, in accordance with a representative embodiment. Specifically, this figure shows an embodiment of the receptacle 2070 of Fig. 20 (or a similar device), with the first housing 2080 of the receptacle 2070 shown in alignment with the second housing 2090 of the receptacle 2070.

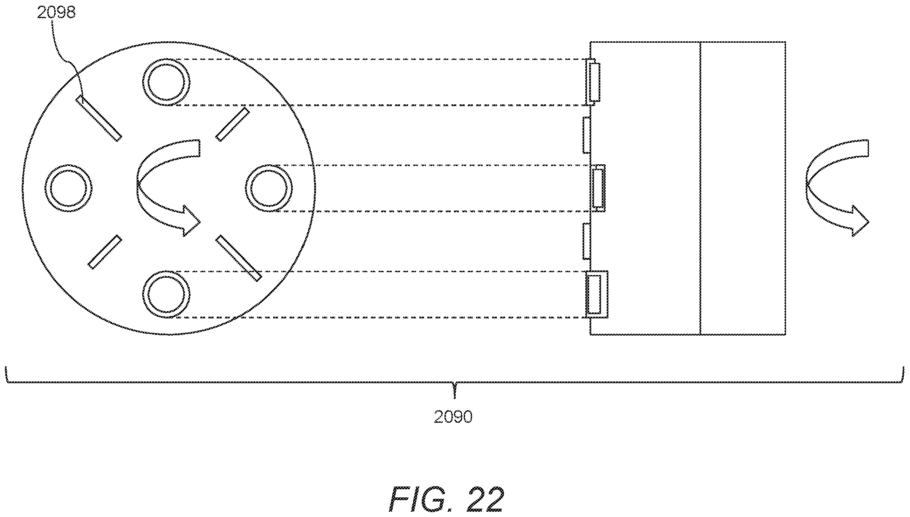

Fig. 22 illustrates a receptacle for collection of a plurality of specimens, in accordance with a representative embodiment. Specifically, this figure shows an embodiment of the receptacle of Fig. 20 (or a similar device), and more specifically a top and side view of the second housing 2090 of such a receptacle. The components 2098 shown in the figure may serve to limit rotation of the second housing 2090 relative to the first housing, e.g., in the direction of the arrows in the figure. These components 2098 may also or instead be structurally configured to act as fixtures on the receptacle that act to sever a portion of a swab based on movement of the housings of the receptacle relative to one another.

The receptacle may be used in a method for collecting a plurality of specimens. The method may include inserting a swab including a shaft and a collection tip into a first bore of a first housing of a receptacle such that at least a portion of the collection tip extends through the first housing and into a second bore of a second housing coupled to the first housing, and moving the second housing relative to the first housing thereby misaligning the first bore from the second bore and severing at least the portion of the collection tip from the swab.

As stated herein, the devices, systems, kits, and methods disclosed herein may be useful in the context of pool testing. However, the devices, systems, kits, and methods disclosed herein may also or instead be useful for other contexts and use cases. For example, a compound swab as disclosed herein may include separable swabs that are separated and then used for different purposes. Continuing with this example, and by way of further example, a first swab of a compound swab could be separated and used for a first analysis, while a second swab of a compound swab could be separated and used for a second analysis that is different from the first analysis (e.g., a different test, and/or a test conducted at a different time and/or place). As such, the swabs of a compound swab could be tested in different labs, and/or they may enter different work streams in one or more labs.

The foregoing description, for purpose of explanation, has been described with reference to specific embodiments. However, the illustrative discussions above are not intended to be exhaustive or to limit the disclosure to the precise forms disclosed. Many modifications and variations are possible in view of the above teachings.

Unless the context clearly requires otherwise, throughout the description, the words "comprise," "comprising," "include," "including," and the like are to be construed in an inclusive sense as opposed to an exclusive or exhaustive sense; that is to say, in a sense of "including, but not limited to." Additionally, the words "herein," "hereunder," "above," "below," and words of similar import refer to this application as a whole and not to any particular portions of this application.

It will be appreciated that the devices, systems, and methods described above are set forth by way of example and not of limitation. Absent an explicit indication to the contrary, the disclosed steps may be modified, supplemented, omitted, and/or re-ordered without departing from the scope of this disclosure. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context.

The method steps of the implementations described herein are intended to include any suitable method of causing such method steps to be performed, consistent with the patentability of the following claims, unless a different meaning is expressly provided or otherwise clear from the context. So, for example performing the step of X includes any suitable method for causing another party such as a remote user, a remote processing resource (e.g., a server or cloud computer) or a machine to perform the step of X. Similarly, performing steps X, Y, and Z may include any method of directing or controlling any combination of such other individuals or resources to perform steps X, Y, and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity, and need not be located within a particular jurisdiction.

It should further be appreciated that the methods above are provided by way of example. The order or presentation of method steps in the description and drawings above is not intended to require this order of performing the recited steps unless a particular order is expressly required or otherwise clear from the context. While particular embodiments have been shown and described, it will be apparent to those skilled in the art that various changes and modifications in form and details may be made therein without departing from the scope of this disclosure and are intended to form a part of the invention as defined by the following claims, which are to be interpreted in the broadest sense allowable by law.

## Claims

1. A method of pool testing, the method comprising:
collecting a specimen using a compound swab (200, 400), the compound swab (200, 400) comprising:
a first swab (210, 410) including a first shaft (212, 412) and a first collection tip (214, 414) disposed on an end of the first shaft (212, 412); and
a second swab (220, 420) coupled to the first swab (210, 410), the second swab (220, 420) including a second shaft (222, 422) and a second collection tip (224, 424) disposed on an end of the second shaft (222, 422);
separating the first swab (210, 410) and the second swab (220, 420);
combining the first swab (210, 410) with a plurality of different swabs for pool testing the specimen with other, different specimens for presence or absence of a component; and
based on results of the pool testing, testing the second swab (220, 420) for presence or absence of the component.

2. The method of claim 1, wherein separating the first swab (210) and the second swab (220) includes disengaging one or more first couplers (331) disposed along the first swab (210) from one or more second couplers (332) disposed along the second swab (220).

3. The method of claim 1, wherein separating the first swab (410) and the second swab (420) includes separating one or more of the first swab (410) and the second swab (420) from a common shaft (402).

4. The method of any of the preceding claims, further comprising labelling the second swab (220, 420).

5. The method of any of the preceding claims, further comprising storing the second swab (220, 420).

6. The method of any of the preceding claims, wherein the testing of the second swab (220, 420) for presence or absence of the component is conducted when pool testing shows that the pool tested positive for the component.

7. The method of any of the preceding claims, wherein the component is the presence of an organism.

8. The method of any of the preceding claims, wherein the component is a clinical marker for disease.

9. The method of any of the preceding claims, wherein the specimen is a nasopharyngeal specimen.

10. The method of any of the preceding claims, wherein collecting the specimen using the compound swab (200, 400) includes inserting the compound swab (200, 400) within an orifice of an animal, and removing the compound swab (200, 400) from the orifice of the animal with portions of the specimen disposed on each of the first collection tip (214, 414) and the second collection tip (224, 424).

11. The method of claim 10, wherein the orifice is a nasal passage of the animal.

12. A system for collecting a specimen for testing, the system comprising:
a compound swab (200, 400) comprising:
a first swab (210, 410) including a first shaft (212, 412) and a first collection tip (212, 412) disposed on an end of the first shaft (212, 412); and
a second swab (220, 420) removably coupled to the first swab (210, 410), the second swab (220, 420) including a second shaft (222, 422) and a second collection tip (224, 424) disposed on an end of the second shaft (222, 422); and
a container (1800) for receiving one or more of the first swab (210, 410) and the second swab (220, 420), the container (1800) comprising:
a first opening (1811) leading to a first chamber (1810), the first chamber (1810) structurally configured to contain at least a portion of one or more of the first swab (210, 410) and the second swab (220, 420) therein; and
a separator (1830) disposed within or adjacent to one or more of the first opening (1811) and the first chamber (1810), the separator (1830) sized and shaped to fit between the first swab (210, 410) and the second swab (220, 420), wherein a predetermined force placed on the compound swab (200, 400) directed toward the container (1800) when the separator (1830) is disposed between the first swab (210, 410) and the second swab (220, 420) separates the first swab (210, 410) and the second swab (220, 420).

13. The system of claim 12, wherein the separator (1830) is located on the container (1800) such that, upon separation of the first swab (210, 410) and the second swab (220, 420) using the separator (1830), one or more of the first swab (210, 410) and the second swab (220, 420) will be at least partially disposed within the first chamber (1810).

14. The system of any of claims 12-13, wherein the container (1800) includes a second opening (1821) leading to a second chamber (1820), and wherein the separator (1830) is disposed between a boundary of the first opening (1811) and the second opening (1821).

15. A method for storing a specimen, the method comprising:
aligning a separator (1830) between a first swab (210, 410) and second swab (220, 420) of a compound swab (200, 400), the separator (1830) coupled to a container (1800) including a first opening (1811) leading to a first chamber (1810), the first chamber (1810) structurally configured to contain at least a portion of one or more of the first swab (210, 410) and the second swab (220, 420) therein; and
applying a predetermined force against the separator (1830) and toward the container (1800) with the compound swab (200, 400) thereby separating the first swab (210, 410) and the second swab (220, 420), wherein at least the portion of one or more of the first swab (210, 410) and the second swab (220, 420) is disposed within the first chamber (1810) upon separation thereof.

## Patentansprüche

1. Pool-Testing-Verfahren, wobei das Verfahren Folgendes umfasst:
Aufnehmen einer Probe unter Verwendung eines zusammengesetzten Tupfers (200, 400), wobei der zusammengesetzte Tupfer (200, 400) Folgendes umfasst:
einen ersten Tupfer (210, 410), der einen ersten Schaft (212, 412) und eine an einem Ende des ersten Schafts (212, 412) angeordnete erste Aufnahmespitze (214, 414) aufweist, und
einen zweiten Tupfer (220, 420), der an den ersten Tupfer (210, 410) gekoppelt ist, wobei der zweite Tupfer (220, 420) einen zweiten Schaft (222, 422) und eine an einem Ende des zweiten Schafts (222, 422) angeordnete zweite Aufnahmespitze (224, 424) aufweist,
Trennen des ersten Tupfers (210, 410) und des zweiten Tupfers (220, 420),
Kombinieren des ersten Tupfers (210, 410) mit einer Vielzahl von verschiedenen Tupfern für das Pool-Testing der Probe mit anderen, verschiedenen Proben auf das Vorliegen oder Nichtvorliegen einer Komponente und
auf der Grundlage von Pool-Testing-Ergebnissen Testen des zweiten Tupfers (220, 420) auf das Vorliegen oder Nichtvorliegen der Komponente.

2. Verfahren nach Anspruch 1, wobei zum Trennen des ersten Tupfers (210) und des zweiten Tupfers (220) das Ausrücken eines oder mehrerer erster Koppler (331), die entlang des ersten Tupfers (210) angeordnet sind, aus einem oder mehreren zweiten Kopplern (332), die entlang des zweiten Tupfers (220) angeordnet sind, gehört.

3. Verfahren nach Anspruch 1, wobei zum Trennen des ersten Tupfers (410) und des zweiten Tupfers (420) das Trennen eines oder mehrerer des ersten Tupfers (410) und des zweiten Tupfers (420) von einem gemeinsamen Schaft (402) gehört.

4. Verfahren nach einem der vorhergehenden Ansprüche, ferner umfassend Etikettieren des zweiten Tupfers (220, 420) .

5. Verfahren nach einem der vorhergehenden Ansprüche, ferner umfassend Aufbewahren des zweiten Tupfers (220, 420) .

6. Verfahren nach einem der vorhergehenden Ansprüche, wobei das Testen des zweiten Tupfers (220, 420) auf Vorliegen oder Nichtvorliegen der Komponente durchgeführt wird, wenn Pool-Testing zeigt, dass der Pool positiv auf die Komponente getestet hat.

7. Verfahren nach einem der vorhergehenden Ansprüche, wobei die Komponente das Vorliegen eines Organismus ist.

8. Verfahren nach einem der vorhergehenden Ansprüche, wobei die Komponente ein klinischer Krankheitsmarker ist.

9. Verfahren nach einem der vorhergehenden Ansprüche, wobei die Probe eine Nasopharyngealprobe ist.

10. Verfahren nach einem der vorhergehenden Ansprüche, wobei die Aufnahme der Probe unter Verwendung des zusammengesetzten Tupfers (200, 400) das Einführen des zusammengesetzten Tupfers (200, 400) in eine Öffnung eines Tiers und Entfernen des zusammengesetzten Tupfers (200, 400) aus der Öffnung des Tiers aufweist, wobei Teile der Probe jeweils auf der ersten Aufnahmespitze (214, 414) und der zweiten Aufnahmespitze (224, 424) angeordnet sind.

11. Verfahren nach Anspruch 10, wobei die Öffnung ein Nasengang des Tiers ist.

12. System zur Aufnahme einer Probe zum Testen, wobei das System Folgendes umfasst:
einen zusammengesetzten Tupfer (200, 400), der Folgendes umfasst:
einen ersten Tupfer (210, 410), der einen ersten Schaft (212, 412) und eine an einem Ende des ersten Schafts (212, 412) angeordnete erste Aufnahmespitze (212, 412) aufweist, und
einen zweiten Tupfer (220, 420), der an den ersten Tupfer (210, 410) gekoppelt ist, wobei der zweite Tupfer (220, 420) einen zweiten Schaft (222, 422) und eine an einem Ende des zweiten Schafts (222, 422) angeordnete zweite Aufnahmespitze (224, 424) aufweist, und
einen Behälter (1800) zum Aufnehmen eines oder mehrerer des ersten Tupfers (210, 410) und des zweiten Tupfers (220, 420), wobei der Behälter (1800) Folgendes umfasst:
eine erste Öffnung (1811), die zu einer ersten Kammer (1810) führt, wobei die erste Kammer (1810) strukturell so ausgestaltet ist, dass sie mindestens einen Teil eines oder mehrerer des ersten Tupfers (210, 410) und des zweiten Tupfers (220, 420) darin enthält, und
eine Trennvorrichtung (1830), die in einer oder mehreren der ersten Öffnung (1811) und der ersten Kammer (1810) oder benachbart dazu angeordnet ist, wobei die Trennvorrichtung (1830) so bemessen und gestaltet ist, dass sie zwischen den ersten Tupfer (210, 410) und den zweiten Tupfer (220, 420) passt, wobei eine auf den zusammengesetzten Tupfer (200, 400) ausgeübte Kraft, die auf den Behälter (1800) zu gerichtet ist, wenn die Trennvorrichtung (1830) zwischen dem ersten Tupfer (210, 410) und dem zweiten Tupfer (220, 420) angeordnet wird, den ersten Tupfer (210, 410) und den zweiten Tupfer (220, 420) trennt.

13. System nach Anspruch 12, wobei die Trennvorrichtung (1830) so an dem Behälter (1800) angeordnet ist, dass bei Trennung des ersten Tupfers (210, 410) und des zweiten Tupfers (220, 420) unter Verwendung der Trennvorrichtung (1830) einer oder mehrere des ersten Tupfers (210, 410) und des zweiten Tupfers (220, 420) mindestens teilweise in der ersten Kammer (1810) angeordnet sind.

14. System nach einem der Ansprüche 12 - 13, wobei der Behälter (1800) eine zweite Öffnung (1821) aufweist, die zu einer zweiten Kammer (1820) führt, und wobei die Trennvorrichtung (1830) zwischen einer Grenze der ersten Öffnung (1811) und der zweiten Öffnung (1821) angeordnet ist.

15. Verfahren zur Aufbewahrung einer Probe, wobei das Verfahren Folgendes umfasst:
Ausrichten einer Trennvorrichtung (1830) zwischen einem ersten Tupfer (210, 410) und einem zweiten Tupfer (220, 420) eines zusammengesetzten Tupfers (200, 400), wobei die an einen Behälter (1800) gekoppelte Trennvorrichtung (1830) eine erste Öffnung (1811) aufweist, die zu einer ersten Kammer (1810) führt, wobei die erste Kammer (1810) strukturell so ausgestaltet ist, dass sie mindestens einen Teil eines oder mehrerer des ersten Tupfers (210, 410) und des zweiten Tupfers (220, 420) darin enthält, und
Ausüben einer vorbestimmten Kraft gegen die Trennvorrichtung (1830) und zu dem Behälter (1800) mit dem zusammengesetzten Tupfer (200, 400) hin, wodurch der erste Tupfer (210, 410) und der zweite Tupfer (220, 420) getrennt werden, wobei mindestens der Teil eines oder mehrerer des ersten Tupfers (210, 410) und des zweiten Tupfers (220, 420) bei deren Trennung in der ersten Kammer (1810) angeordnet ist.

## Revendications

1. Procédé de test groupé, le procédé comprenant :
la collecte d'un échantillon au moyen d'un écouvillon multiple (200, 400), l'écouvillon multiple (200, 400) comprenant :
un premier écouvillon (210, 410) comportant une première tige (212, 412) et un premier bout de collecte (214, 414) disposé sur une extrémité de la première tige (212, 412) ; et
un deuxième écouvillon (220, 420) couplé au premier écouvillon (210, 410), le deuxième écouvillon (220, 420) comportant une deuxième tige (222, 422) et un deuxième bout de collecte (224, 424) disposé sur une extrémité de la deuxième tige (222, 422) ;
la séparation du premier écouvillon (210, 410) et du deuxième écouvillon (220, 420) ;
la combinaison du premier écouvillon (210, 410) avec une pluralité d'écouvillons différents pour un test groupé de l'échantillon avec d'autres échantillons, différents, pour la présence ou l'absence d'un composant ; et
sur la base des résultats du test groupé, le test du deuxième écouvillon (220, 420) pour la présence ou l'absence du composant.

2. Procédé selon la revendication 1, dans lequel la séparation du premier écouvillon (210) et du deuxième écouvillon (220) comporte le désaccouplement d'un ou plusieurs premiers dispositifs de couplage (331) disposés le long du premier écouvillon (210) d'un ou plusieurs deuxièmes dispositifs de couplage (332) disposés le long du deuxième écouvillon (220).

3. Procédé selon la revendication 1, dans lequel la séparation du premier écouvillon (410) et du deuxième écouvillon (420) comporte la séparation du premier écouvillon (410) et/ou du deuxième écouvillon (420) d'une tige commune (402).

4. Procédé selon l'une quelconque des revendications précédentes, comprenant en outre l'étiquetage du deuxième écouvillon (220, 420).

5. Procédé selon l'une quelconque des revendications précédentes, comprenant en outre le stockage du deuxième écouvillon (220, 420).

6. Procédé selon l'une quelconque des revendications précédentes, dans lequel le test du deuxième écouvillon (220, 420) pour la présence ou l'absence du composant est conduit quand le test groupé montre que le groupe a été testé positif pour le composant.

7. Procédé selon l'une quelconque des revendications précédentes, dans lequel le composant est la présence d'un organisme.

8. Procédé selon l'une quelconque des revendications précédentes, dans lequel le composant est un marqueur clinique d'une maladie.

9. Procédé selon l'une quelconque des revendications précédentes, dans lequel l'échantillon est un échantillon nasopharyngé.

10. Procédé selon l'une quelconque des revendications précédentes, dans lequel la collecte de l'échantillon au moyen de l'écouvillon multiple (200, 400) comporte l'insertion de l'écouvillon multiple (200, 400) à l'intérieur d'un orifice d'un animal, et le retrait de l'écouvillon multiple (200, 400) de l'orifice de l'animal avec des parties de l'échantillon disposées à la fois sur le premier bout de collecte (214, 414) et le deuxième bout de collecte (224, 424).

11. Procédé selon la revendication 10, dans lequel l'orifice est une voie nasale de l'animal.

12. Système destiné à collecter un échantillon à tester, le système comprenant :
un écouvillon multiple (200, 400) comprenant :
un premier écouvillon (210, 410) comportant une première tige (212, 412) et un premier bout de collecte (214, 414) disposé sur une extrémité de la première tige (212, 412) ; et
un deuxième écouvillon (220, 420) couplé de façon amovible au premier écouvillon (210, 410), le deuxième écouvillon (220, 420) comportant une deuxième tige (222, 422) et un deuxième bout de collecte (224, 424) disposé sur une extrémité de la deuxième tige (222, 422) ; et
un récipient (1800) destiné à recevoir le premier écouvillon (210, 410) et/ou le deuxième écouvillon (220, 420), le récipient (1800) comprenant :
une première ouverture (1811) conduisant à une première chambre (1810), la première chambre (1810) étant structuralement configurée pour contenir en son sein au moins une partie du premier écouvillon (210, 410) et/ou du deuxième écouvillon (220, 420) ; et
un séparateur (1830) disposé à l'intérieur ou au voisinage de la première ouverture (1811) et/ou de la première chambre (1810), le séparateur (1830) étant dimensionné et façonné pour s'ajuster entre le premier écouvillon (210, 410) et le deuxième écouvillon (220, 420), une force prédéterminée appliquée sur l'écouvillon multiple (200, 400) dirigée vers le récipient (1800) quand le séparateur (1830) est disposé entre le premier écouvillon (210, 410) et le deuxième écouvillon (220, 420) séparant le premier écouvillon (210, 410) et le deuxième écouvillon (220, 420).

13. Système selon la revendication 12, dans lequel le séparateur (1830) est situé sur le récipient (1800) de telle sorte que, lors de la séparation du premier écouvillon (210, 410) et du deuxième écouvillon (220, 420) au moyen du séparateur (1830), le premier écouvillon (210, 410) et/ou le deuxième écouvillon (220, 420) seront au moins partiellement disposés à l'intérieur de la première chambre (1810).

14. Système selon l'une quelconque des revendications 12 et 13, dans lequel le récipient (1800) comporte une deuxième ouverture (1821) conduisant à une deuxième chambre (1820), et dans lequel le séparateur (1830) est disposé entre une limite de la première ouverture (1811) et la deuxième ouverture (1821).

15. Procédé de stockage d'un échantillon, le procédé comprenant :
l'alignement d'un séparateur (1830) entre un premier écouvillon (210, 410) et un deuxième écouvillon (220, 420) d'un écouvillon multiple (200, 400), le séparateur (1830) étant couplé à un récipient (1800) comportant une première ouverture (1811) conduisant à une première chambre (1810), la première chambre (1810) étant structuralement configurée pour contenir en son sein au moins une partie du premier écouvillon (210, 410) et/ou du deuxième écouvillon (220, 420) ; et
l'application d'une force prédéterminée sur le séparateur (1830) et vers le récipient (1800) avec l'écouvillon multiple (200, 400), ce faisant la séparation du premier écouvillon (210, 410) et du deuxième écouvillon (220, 420), au moins la partie du premier écouvillon (210, 410) et/ou du deuxième écouvillon (220, 420) étant disposée à l'intérieur de la première chambre (1810) lors de la séparation de ceux-ci.
